(19) Europäisches Patentamt
European Patent Office
Office européen des brevets

(11) **EP 3 332 689 B1**

(12) **EUROPEAN PATENT SPECIFICATION**

(45) Date of publication and mention
of the grant of the patent:
**09.12.2020 Bulletin 2020/50**

(21) Application number: **16832412.7**

(22) Date of filing: **06.09.2016**

(51) Int Cl.:
*A61B 1/04* *(2006.01)*          *A61B 5/00* *(2006.01)*
*A61K 49/00* *(2006.01)*          *G06T 7/12* *(2017.01)*
*G06T 7/174* *(2017.01)*          *G06T 7/37* *(2017.01)*

(86) International application number:
**PCT/IB2016/055318**

(87) International publication number:
**WO 2017/021942 (09.02.2017 Gazette 2017/06)**

(54) **SYSTEM FOR ALIGNING MULTIPLE FLUORESCENT IMAGES COMPRISING A PEN-SHAPED IMAGING DEVICE WITH A PLURALITY OF LIGHT SOURCES**

SYSTEM ZUR AUSRICHTUNG MEHRERER FLUORESZENZBILDER UMFASSEND EIN STIFTFÖRMIGES BILDGEBUNGSGERÄT MIT EINER MEHRZAHL VON LICHTQUELLEN

SYSTÈME POUR ALIGNER DE MULTIPLES IMAGES FLUORESCENTES COMPRENANT UN DISPOSITIF D'IMAGERIE EN FORME DE STYLO AVEC UNE PLURALITÉ DE SOURCES DE LUMIÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.08.2015 KR 20150109516**

(43) Date of publication of application:
**13.06.2018 Bulletin 2018/24**

(73) Proprietor: **National Cancer Center**
**Goyang-si, Gyeonggi-do 10408 (KR)**

(72) Inventors:
• **LEE, Seung Hoon**
**Seoul 01798 (KR)**
• **KIM, Kwang Gi**
**Seoul 05503 (KR)**

• **YOO, Heon**
**Seoul 06288 (KR)**

(74) Representative: **Schiweck Weinzierl Koch Patentanwälte Partnerschaft mbB Ganghoferstraße 68 B 80339 München (DE)**

(56) References cited:
EP-A1- 2 422 688          WO-A1-2009/028136
JP-A- 2012 005 807        KR-A- 20120 092 573
US-A1- 2006 241 499       US-A1- 2008 049 314
US-A1- 2011 026 786       US-A1- 2011 184 243
US-A1- 2012 294 537

Remarks:
The filing date of the international application is within two months from the date of expiration of the priority period (R. 26bis.3 PCT).

**Description**

BACKGROUND

**1. Field of the Invention**

The present invention relates to a system for aligning multiple fluorescent images, the system including a medical fluorescent imaging device.

**2. Discussion of Related Art**

In general, various types of video equipment are being used to obtain biomedical imaging information at biological and medical sites. In such video equipment, in comparison to other imaging techniques, a biomedical imaging method employing light is widely being used due to its convenience of providing real-time information to an observer or a surgeon. The document US 2011/184243 A1 discloses a medical fluorescent imaging device with a probe including first and second selectively controllable light sources.

In addition, the rapid development of molecular imaging technology lately has made it possible to diagnose diseases and detect lesions. In particular, nuclear medicine imaging and magnetic resonance imaging (MRI) technology is attracting attention as examination systems that are very useful for diagnosing diseases. MRI technology is a method of obtaining anatomical, physiological, and biochemical information images of a body by using a phenomenon in which the spinning of hydrogen atoms is relaxed. MRI technology is an excellent diagnostic imaging technology that is not invasive towards body organs of a person or an animal and makes it possible to capture images thereof in real time.

However, in the diagnosis of a malignant glioma that is highly infiltrative, there are problems with such MRI in that it is difficult to determine the boundary between a malignant glioma and normal tissue.

During surgical operation on a tumor, it is necessary to check the positions and connections of cerebral blood vessels as well as the tumor for the safety of the patient. In particular, to increase the survival rate of patients and prevent recurrences of diseases, it is very important to completely remove a malignant tumor or a tumor and minimize damage to normal tissue by minimizing the removed parts.

Relevant to this, 5-aminolevulinic acid (5-ALA), which is a fluorescent luminescent material for marking tumors and blood vessels, is injected into the human body and then fluoresces due to generation of protoporphyrin IX (PpIX) derived from a material transformation process caused by metabolism in the body. With such a reaction process, 5-ALA serves as a target fluorescence marker targeting only cancer cells.

Also, indocyanine green (ICG), which is a fluorescent luminescent material for marking blood vessels, marks blood vessels and lymph nodes while circulating through the blood vessels and the lymph nodes.

In other words, 5-ALA is used to cause cancer cells to fluoresce, and ICG is used to cause blood vessels to fluoresce.

However, surgical fluorescent imaging equipment for surgical microscopes may display only one type of fluorescent image due to the presence of one type of fluorescent luminescent material, and thus displays only ICG fluorescent images. Also, since fluorescent imaging equipment is very large, there is an inconvenience in terms of using fluorescent imaging equipment only for surgery.

Consequently, an apparatus for accurately detecting and showing the position of a glioma and the positions of blood vessels at the same time is required for a patient's safety and ease of surgery.

SUMMARY OF THE INVENTION

The present invention is directed to a system for acquiring multiple fluorescent images, the system comprising a pen-shaped medical fluorescent imaging device and being capable of detecting and showing the positions of blood vessels and the position of a glioma at the same time for a patient's safety and ease of surgery.

The system for acquiring multiple fluorescent images makes it possible for an operator to accurately and easily check the positions and connections of a tumor and blood vessels by acquiring multiple fluorescent images in which the positions of the blood vessels and the boundary of a glioma are marked to luminesce due to multiple fluorescent materials, and by aligning and displaying the acquired multiple fluorescent images in real time.

The pen-type medical fluorescent imaging device comprises: a probe configured to extend in a longitudinal direction and include an image capturing section at one end; a plurality of light sources configured to be arranged to surround the image capturing section; and a controller configured to control the light sources. The light sources include a first light source configured to emit light of a first wavelength range so that blood vessels are marked by a first fluorescent material, and a second light source configured to emit light of a second wavelength range so that a glioma is marked by a second fluorescent material. The first and second light sources are selectively controlled by the controller.

Furthermore, a system for aligning multiple fluorescent images is provided, the system including: the pen-type medical fluorescent imaging device; a fluorescent multi-image acquirer configured to acquire an actual image of a target part, a fluorescent vascular image in which blood vessels are marked by the first fluorescent material, and a fluorescent glioma image in which a glioma is marked by the second fluorescent material; a blood vessel extractor configured to extract a vascular shape from the acquired fluorescent vascular image; a glioma image processor configured to process the acquired fluorescent glioma image so that a boundary of the glioma becomes distinct; a fluorescent multi-image aligner configured to align the extracted fluorescent vascular image and fluorescent glioma image in real time; and a fluorescent image display configured to display a fluorescent multi-image in which the fluorescent vascular image and the fluorescent glioma image are aligned.

BRIEF DESCRIPTION OF THE DRAWINGS

The above description and other objects, features and advantages of the present invention will become more apparent to those of ordinary skill in the art by describing in detail exemplary embodiments thereof with reference to the accompanying drawings, in which:

FIG. 1 is a perspective view of a pen-type medical fluorescent imaging device;
FIG. 2 is a front view of the pen-type medical fluorescent imaging device;
FIG. 3 is a conceptual diagram for facilitating understanding of the overall operation of a system for aligning multiple fluorescent images; and
FIG. 4 is a block diagram of the system for aligning multiple fluorescent images.

DETAILED DESCRIPTION OF EXEMPLARY EMBODIMENTS

A pen-type medical fluorescent imaging device is disclosed that is capable of detecting and showing the position of a glioma and the positions of blood vessels at the same time for a patient's safety and ease of surgery.

Here, a pen type denotes a small pen shape that may be conveniently and freely used with a hand like a pen, which is a writing instrument. The probe 110 is here formed in a pen shape.

The pen-type medical fluorescent imaging device which is part of the present invention includes: a probe that extends in a longitudinal direction and includes an image capturing section at one end; a plurality of light sources that are arranged to surround the image capturing section; and a controller that controls the light sources. The light sources include a first light source that emits light of a first wavelength range so that blood vessels are marked by a first fluorescent material, and a second light source that emits light of a second wavelength range so that a glioma is marked by a second fluorescent material. The first and second light sources are selectively controlled by the controller.

Meanwhile, the light sources may include filters. The first light source may include a first filter, and the second light source may include a second filter.

Here, the first fluorescent material may include indocyanine green (ICG), and the second fluorescent material may include one of 5-aminolevulinic acid (5-ALA) and protoporphyrin IX (PpIX) converted from 5-ALA. The light sources may include a third light source that emits white light.

Also, the first wavelength range may be 750 nm to 800 nm, and the second wavelength range may be 350 nm to 450 nm. The probe is formed of a flexible material.

In addition, the present invention relates to a system for acquiring multiple fluorescent images. The system includes: the pen-type medical fluorescent imaging device; a fluorescent multi-image acquirer that acquires an actual image of a target part, a fluorescent vascular image in which blood vessels are marked by the first fluorescent material, and a fluorescent glioma image in which a glioma is marked by the second fluorescent material; a blood vessel extractor that extracts a vascular shape from the acquired fluorescent vascular image; a glioma image processor that processes the acquired fluorescent glioma image so that the boundary of the glioma becomes distinct; a fluorescent multi-image aligner that aligns the extracted fluorescent vascular image and the fluorescent glioma image in real time; and a fluorescent image display that displays a fluorescent multi-image in which the fluorescent vascular image and the fluorescent glioma image are aligned.

Here, the fluorescent multi-image acquirer includes a fluorescent vascular shape image acquirer that acquires the fluorescent vascular image in which the positions of the blood vessels are marked by the first fluorescent material, and a fluorescent glioma image acquirer that acquires the fluorescent glioma image in which the position of the glioma is marked by the second fluorescent material.

In addition, the fluorescent multi-image acquirer may be configured to acquire transformation parameters corresponding to the highest similarity by measuring pixel value-based similarities of a moving image with respect to the actual image, and match the moving image to the actual image by performing two-dimensional (2D) centered similarity registration based on the acquired transformation parameters so that coordinate errors between the actual image and the moving image are minimized, and the moving image may be one of the fluorescent vascular image and the fluorescent glioma image.

The fluorescent image display may display at least one of the actual image, the fluorescent vascular image, the fluorescent glioma image, and the fluorescent multi-image according to a selection of an operator or a user. The fluorescent image display may set individual opacity values of the actual image, the fluorescent vascular image, the fluorescent glioma image, and the fluorescent multi-image to simultaneously display a plurality of images overlapping with each other, and may adjust the opacity values of the individual images to display only a specific image as necessary.

The pen-type medical fluorescent imaging device may include a transmitter capable of transmitting the actual image, the fluorescent vascular image, and the fluorescent glioma image captured by the image capturing section to the fluorescent multi-image acquirer through wireless communication.

Hereinafter, exemplary embodiments will be described in detail with reference to the accompanying drawings.

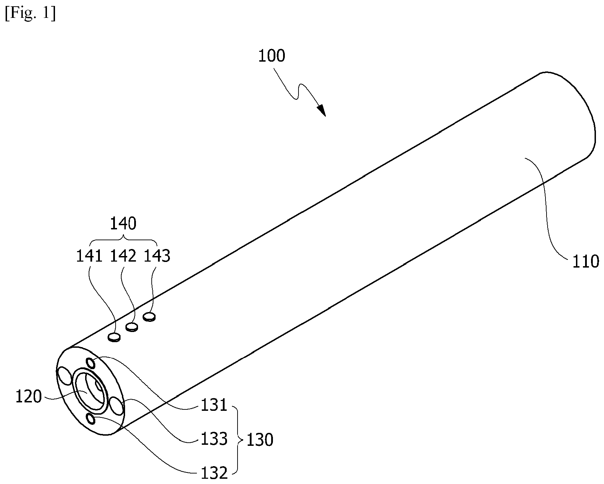

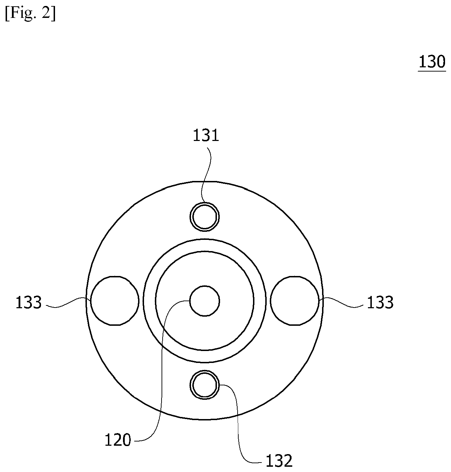

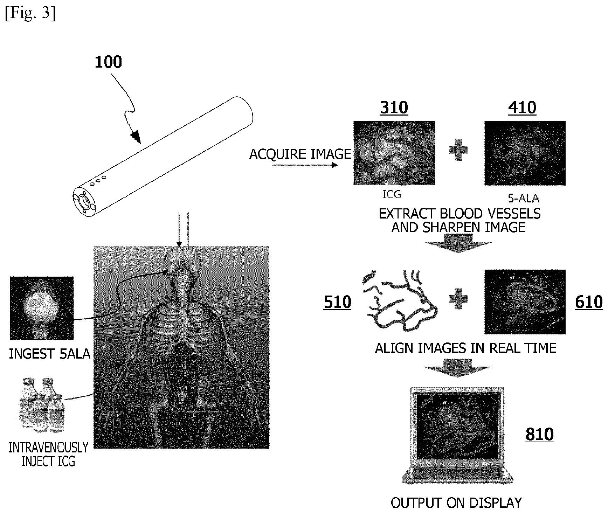

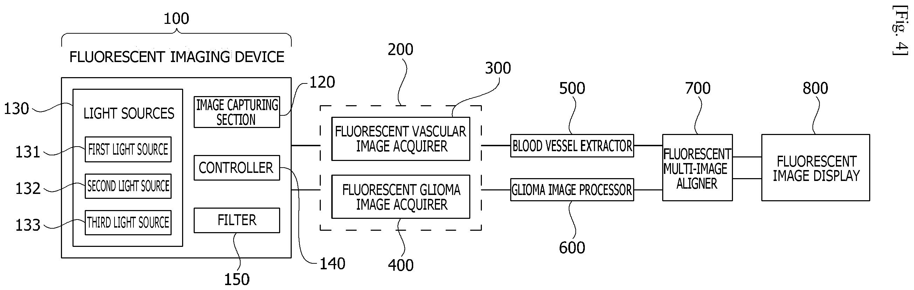

FIG. 1 is a perspective view of a pen-type medical fluorescent imaging device, FIG. 2 is a front view of the pen-type medical fluorescent imaging device, FIG. 3 is a conceptual diagram for facilitating understanding of overall operation of a system for aligning multiple fluorescent images, and FIG. 4 is a block diagram of the system for aligning multiple fluorescent images. A pen-type medical fluorescent imaging device and a system for acquiring multiple fluorescent images will be described below with reference to FIGS. 1 to 4 and exemplary embodiments.

A pen-type medical fluorescent imaging device 100 is provided that is capable of detecting and showing the positions of blood vessels and the position of a glioma at the same time for a patient's safety and ease of surgery.

As shown in FIGS. 1 and 2, the medical fluorescent imaging device 100 is formed as a pen type, and includes the probe 110 extending in a longitudinal direction and an image capturing section 120 and light sources 130 at one end of the probe 110.

Here, a pen type denotes a small pen shape that may be conveniently and freely used with a hand like a pen, which is a writing instrument. The probe 110 is here formed in a pen shape.

The probe 110 may include the image capturing section 120 and the light sources 130 at the end, and the light sources 130 may be plural in number to surround the image capturing section 120. Here, the light sources 130 may include a first light source 131 and a second light source 132. The first light source 131 may emit light of a first wavelength range so that blood vessels are marked by a first fluorescent material, and the second light source 132 may emit light of a second wavelength range so that a glioma is marked by a second fluorescent material. For reference, the light sources 130 may be light-emitting diode (LED) lamps, and may preferably be near-infrared LED lamps. The number of LED lamps that emit light may be adjusted according to the size and shape of a target part.

The image capturing section 120 captures an image of a target part, and may be a general camera for capturing an actual image of a target part, a fluorescent vascular image in which blood vessels are marked by the first fluorescent material, and a fluorescent glioma image in which a glioma is marked by the second fluorescent material.

In particular, a digital camera or a near infrared ray camera capable of capturing and outputting or storing an image in real time may be used as the image capturing section 120. For example, a charge coupled device (CCD) or complementary metal-oxide semiconductor (CMOS) camera may be used, and a CCD or CMOS camera having a resolution of megapixel level or greater may preferably be used. Such a camera with megapixel level resolution or greater may be used to acquire a vascular and/or glioma image at high resolution and to observe blood vessels in real time (it is possible to acquire images of 30 frames or more per second). Therefore, convenience for doctors and patients is improved, and it is possible to rapidly and easily observe blood vessels and/or a glioma during a medical examination and surgery of a patient.

In particular, the light sources 130 and the image capturing section 120 are installed at a front end of the probe 110 such that loss of light and image quality may be reduced.

In addition, the fluorescent imaging device 100 may include a controller 140 that controls the light sources 130. The first light source 131 and the second light source 132 may be selectively controlled by the controller 140 to emit light. The controller 140 may be a conventional switch, and may be supplied with current from a power supply and may turn on or off the light sources 130. Meanwhile, the controller 140 that controls the first light source 131 and the second light source 132 may be a first switch 141 and a second switch 142.

In the present case, ICG and also PpIX, which are near-infrared fluorescent materials, may be used as fluorescent materials, PpIX converted from 5-ALA. The first fluorescent material may be ICG, and the second fluorescent material may be 5-ALA.

In particular, ICG, which is efficient in terms of detecting the positions of a patient's blood vessels and lymph nodes, is a fluorescent material that has been used since 1957 and has few side effects. When injected into a patient, ICG is combined with plasma protein, and when light in a range of 750 nm to 800 nm is transmitted, ICG radiates fluorescent light having a fluorescence peak value of 845 nm.

5-ALA, which is efficient in terms of detecting the position of a glioma, is converted in cells to PpIX which has a fluorescence peak value of 635 nm and radiates fluorescent light when light of about 400 nm is transmitted to PpIX converted in cells (it is also possible to use radiated light of about 405 nm). Using this property, it is possible to distinguish between normal tissue and a malignant glioma. The use of 5-ALA increases a success rate of complete removal of a malignant glioma about 1.4 times, and reduces a malignant glioma which is not removed to 1/16 of its size, and is thus effective in terms of preventing recurrence of the malignant glioma.

Meanwhile, the medical fluorescent imaging device 100 may emit light of various wavelengths through the light sources 130, and a laser, an LED may be used as light sources. The light sources 130 may include a filter 150, which provides an excitation wavelength band at which multiple fluorescent materials distributed in a target body such as a human body are excited and provides a light-emitting wavelength band at which light is emitted from the excited multiple fluorescent materials. The filter 150 may provide an excitation wavelength band and a light-emitting wavelength band that are different from each other.

More specifically, the first light source 131 may include a first filter, and the second light source 132 may include a second filter.

In particular, after ICG is intravenously injected into a patient and 5-ALA is ingested by the patient, light in a range of 750 nm to 800 nm may be transmitted to a treatment area of the patient through the first light source of the medical fluorescent imaging device 100. Then, ICG combined with plasma protein in the target body may radiate fluorescent light having a peak value of 845 nm.

Also, when the second light source 132 transmits light of 400 nm to the treatment area including PpIX converted from 5-ALA through metabolism in cells, fluorescent light having a peak value of 635 nm may be radiated by PpIX distributed in a glioma.

In other words, the first wavelength range may be 750 nm to 800 nm, and the second wavelength range may be 350 nm to 450 nm.

In addition, the medical fluorescent imaging device 100 may include a third light source 133 that may radiate white light. The controller 140 for controlling the third light source 133 may be a third switch 143.

More specifically, the third light source 133 may be used in diagnosis of a patient by a doctor, and medical personnel may use the third light source 133 as a substitute for a medical pen light.

Here, a pen light is used by a doctor to diagnose a patient. As a light formed in a pen shape, a pen light enables a user to conveniently carry it around or turn it on in the dark to identify objects.

The medical fluorescent imaging device 100 may include a battery for power supply that is installed so as to be replaceable in the probe 110. In addition, a clip (not shown) for fixing the medical fluorescent imaging device 100 to a doctor's gown may be included.

The medical fluorescent imaging device 100 having such a configuration including the third light source 133 may be used in a doctor's office, a ward to emit light on dim areas of a patient's body such as the mouth, nose, ears, that natural light, general lighting, cannot reach and to examine an affected area or used to emit light to an eye and check a pupillary reflex.

Meanwhile, the probe 110 may be formed of a flexible material. In this case, a user may change the position of a total cross section irradiated by the light sources 130 as necessary, and may easily emit light to a part to be examined and capture an image of the part, such that the convenience and accuracy of work are improved. The probe 110 may be formed of a metal or a synthetic resin such as polypropylene (PP), polyethylene (PE), nylon, and a bellow pipe may be formed as a particular aspect.

Here, any flexible material fulfilling such a purpose may be used for the probe 110.

A system for aligning multiple fluorescent images is disclosed which makes it possible for an operator to accurately and easily check the positions and connections of a tumor and blood vessels by acquiring multiple fluorescent images in which the positions of the blood vessels and the boundary of a glioma are marked to luminesce due to multiple fluorescent materials, and by aligning and displaying the acquired multiple fluorescent images in real time.

More specifically, referring to FIGS. 3 and 4, the system for aligning multiple fluorescent images includes the pen-type medical fluorescent imaging device 100, a fluorescent multi-image acquirer 200 that acquires an actual image of a target part, a fluorescent vascular image 310 in which blood vessels are marked by the first fluorescent material, and a fluorescent glioma image 410 in which a glioma is marked by the second fluorescent material, a blood vessel extractor 500 that extracts vascular shapes from the acquired fluorescent vascular image 310, a glioma image processor 600 that processes the acquired fluorescent glioma image 410 so that the boundary of the glioma becomes distinct, a fluorescent multi-image aligner 700 that aligns an extracted vascular image 510 of the blood vessel extractor 500 and a processed glioma image 610 in real time, and a fluorescent image display 800 that displays a fluorescent multi-image in which the extracted vascular image 510 and the processed glioma image 610 are aligned.

Here, the fluorescent multi-image acquirer 200 includes a fluorescent vascular image acquirer 300 that acquires the fluorescent vascular image 310 in which the positions of blood vessels are marked by the first fluorescent material, and a fluorescent glioma image acquirer 400 that acquires the fluorescent glioma image 410 in which the position of a glioma is marked by the second fluorescent material.

Meanwhile, the medical fluorescent imaging device 100 additionally includes a transmitter capable of transmitting the actual image, the vascular image, and the glioma image captured by the image capturing section 120 from the medical fluorescent imaging device 100 to the fluorescent multi-image acquirer 200 through wireless communication.

The transmitter may transmit images captured by the image capturing section 120 as well as the fluorescent multi-image acquirer 200 to devices capable of monitoring the images such as a computer, a smart phone. Doctors may check images captured in real time as required by using their smart phones. In this case, images may be transmitted through wireless Internet, Bluetooth.

The fluorescent multi-image aligner 700 acquires transformation parameters corresponding to the highest similarity by measuring pixel value-based similarities of moving images with respect to a fixed image to perform 2D centered similarity registration, and matches moving images to the fixed image by using normalized cross-correlation (NCC).

A fixed image denotes the actual image, and moving images denote the acquired fluorescent vascular image 310 and the acquired fluorescent glioma image 410.

Meanwhile, the present system may display one or more of the actual image, the fluorescent vascular image 310, and the fluorescent glioma image 410 captured by the image capturing section 120 and a fluorescent multi-image 810 according to a selection of an operator or a user. The display 800 may simultaneously display a plurality of images to overlap with each other by separately setting the opacity of the actual image, the fluorescent vascular image 310, the fluorescent glioma image 410, and the fluorescent multi-image 810, and may display only specific images as necessary by adjusting an opacity value of each image.

A method of aligning multiple fluorescent images by using the system for aligning multiple fluorescent images including the pen-type medical fluorescent imaging device 100 will be described in detail below with reference to accompanying drawings.

First, ICG is intravenously injected into a patient, and 5-ALA is ingested by the patient. After 5-ALA is ingested, the time for a sufficient amount of PpIX to accumulate in tumor tissue is preferably necessary before excitation light is emitted to excite PpIX. Specifically, it may take four to eight hours.

Subsequently, when light in a range of 750 nm to 800 nm is transmitted to a treatment area of the patient through the first light source 131 of the medical fluorescent imaging device 100, ICG combined with plasma protein in the target body may radiate fluorescent light having a peak value of 845 nm. The fluorescent vascular image acquirer 300 may acquire the fluorescent vascular image 310 in which blood vessels radiate fluorescent light of 845 nm due to ICG combined with plasma protein.

Also, when the second light source 132 transmits light of about 400 nm to the treatment area including PpIX converted from 5-ALA through metabolism in cells, it is possible to acquire the fluorescent glioma image 410 in which a glioma radiates fluorescent light having a peak value of 635 nm due to PpIX distributed to the glioma.

Then, vascular positions are marked with fluorescence due to ICG as shown in the fluorescent vascular image 310 of FIG. 3, and also the position of the glioma is marked with fluorescence due to PpIX converted from 5-ALA as shown in the fluorescent glioma image 410.

Here, the blood vessel extractor 500 may extract vascular shapes from the fluorescent vascular image 310, and the vascular shapes may be extracted from the ICG fluorescent vascular image 310 by using histogram equalization and Otsu thresholding, which is an automatic binarization technique using a histogram.

The glioma image processor 600 may process the fluorescent glioma image 410 so that the position and the boundary of the glioma become more distinct.

For reference, the extracted vascular image 510, excluding the blood vessels marked with fluorescence, may be converted into a grey color and then used for image alignment based on feature points (edges, line parts). Also, the processed glioma image 610, excluding the glioma, may be converted into a grey color and then used for image alignment.

The fluorescent multi-image aligner 700 may perform image matching on even a fluorescent image that shows morphology slightly different from the color values of the actual image, and may align and match, as a functional image, two or three such images to each other to show different characteristics based on wavelength range. Therefore, it is possible for the operator to see a fluorescent image in which the positions of a glioma and blood vessels are distinct.

The fluorescent multi-image aligner 700 performs 2D centered similarity registration to minimize coordinate errors between the actual image and an infrared (IR) image (the extracted vascular image 510 or the processed glioma image 610). As shown in FIG. 3, the fluorescent multi-image aligner 700 acquires transformation parameters corresponding to the highest similarity by measuring a pixel value-based similarity of a moving image (the vascular shape image or the fluorescent glioma image) with respect to a fixed image (the actual image) to perform 2D centered similarity registration, and matches the moving image to the fixed image by using NCC.

The fluorescent multi-image aligner 700 measures a pixel value-based similarity of the fluorescent vascular image 310 with respect to the actual image and continuously changes the fluorescent vascular image 310 until a preset condition is satisfied. When the preset condition is not satisfied within the maximum number of interpolations, transformation parameters corresponding to a highest similarity are acquired.

A total of six parameters may be acquired through similarity registration: a scale factor, a radian-based angle, x and y values of a center position (x, y) of a moving image after transformation, and translated x and y values (x', y').

The six transformation parameters acquired through similarity registration are used to transform a vascular image to coincide with the actual image using Equation 1. In other words, similarity conversion parameters for rotation, translation, and uniform scaling of an ICG image (the fluorescent vascular image 310) are calculated with respect to the actual image that is a color image, and the calculated parameters are applied to the ICG image for resampling. Then, an image matching section matches the ICG image to the actual image.

[Equation 1]

$$\begin{bmatrix} x' \\ y' \end{bmatrix} = \begin{bmatrix} \lambda & 0 \\ 0 & \lambda \end{bmatrix} \begin{bmatrix} \cos\theta & -\sin\theta \\ \sin\theta & \cos\theta \end{bmatrix} \begin{bmatrix} x - C_x \\ y - C_y \end{bmatrix} + \begin{bmatrix} T_x + C_x \\ T_y + C_y \end{bmatrix}$$

In Equation 1, $\lambda$ is a scale factor, $\theta$ is a rotation angle, $(C_x, C_y)$ are values of a rotation center position, and $(T_x, T_y)$ are values of translated elements.

After a differential image is generated, similarity is calculated by using NCC as shown in Equation 2. Since the images have different colors, no pixel value-based image is generated. Rather, differential values of the images are used, and then a normalized correlation value is used to perform image matching.

[Equation 2]

$$R(x,y) = \frac{\sum_{x',y'} (T'(x',y') \cdot I'(x+x',y+y'))}{\sqrt{\sum_{x',y'} T'(x',y')^2} \sqrt{\sum_{x',y'} I'(x+x',y+y')^2}}$$

In Equation 2, R(x, y) denotes a calculated similarity value, T denotes a target image, and I denotes an original image.

The fluorescent vascular image 310 and the fluorescent glioma image 410 are aligned by the fluorescent multi-image aligner 700 so that different sets of data are transformed into one coordinate system.

The fluorescent image display 800 displays, to the operator, an image in which both the positions of blood vessels and the boundary of the glioma aligned by the fluorescent multi-image aligner 700 are distinctly marked. The fluorescent image display 800 displays at least one of the actual image of the treatment area, the fluorescent vascular image 310 in which blood vessels are marked to fluoresce due to ICG, the fluorescent glioma image 410 in which the boundary of a glioma is marked to fluoresce based on 5-ALA, and a fluorescent multi-image in which the fluorescent vascular image 310 and the fluorescent glioma image 410 are aligned according to a selection of the operator or a user.

Also, the fluorescent image display 800 may set an opacity value of each image and simultaneously display a plurality of images overlapping with each other, or may adjust the opacity value of each image and display only a specific image as necessary.

Due to such operations, it is possible to align, as one image, the fluorescent vascular image 310 in which the positions of blood vessels are marked due to ICG and the fluorescent glioma image 410 in which the boundary of the glioma is marked based on 5-ALA, and it is possible to provide the operator with an image in which both the positions of blood vessels and the boundary of the glioma are distinctly marked.

Since the pen-type medical fluorescent imaging device includes both a first light source and a second light source for showing the positions of both a glioma and blood vessels, it is possible to selectively know the positions of the blood vessels and the glioma.

Since the pen-type medical fluorescent imaging device is miniaturized in the form of a probe, it can be easily held by an operator and carried around. Also, it is possible to reduce loss of light and image quality by installing light sources and an image capturing section at a front end.

The system for aligning multiple fluorescent images makes it possible for an operator to accurately and easily check the positions and connections of a tumor and blood vessels by acquiring multiple fluorescent images in which the positions of the blood vessels and the boundary of a glioma are marked to luminesce due to multiple fluorescent materials having different properties in a target part, and by aligning and displaying the acquired multiple fluorescent images in real time.

It will be apparent to those skilled in the art that various modifications can be made to the above-described exemplary embodiments.

The invention is defined by the appended claims.

< DESCRIPTION OF REFERENCE NUMERALS>

100: FLUORESCENT IMAGING DEVICE
120: IMAGE CAPTURING SECTION
130: LIGHT SOURCES
131: FIRST LIGHT SOURCE
132: SECOND LIGHT SOURCE
133: THIRD LIGHT SOURCE
140: CONTROLLER
150: FILTER
300: FLUORESCENT VASCULAR IMAGE ACQUIRER
400: FLUORESCENT GLIOMA IMAGE ACQUIRER
500: BLOOD VESSEL EXTRACTOR
600: GLIOMA IMAGE PROCESSOR
700: FLUORESCENT MULTI-IMAGE ALIGNER
800: FLUORESCENT IMAGE DISPLAY

**Claims**

1. A system for acquiring multiple fluorescent images, the system comprising:

a pen-shaped medical fluorescent imaging device (100) comprising a probe (110) configured to extend in a longitudinal direction and include an image capturing section (120) at one end; a plurality of light sources (130, 131, 132, 133) configured to be arranged to surround the image capturing section (120); and a controller (140) configured to control the light sources (130, 131, 132, 133), wherein the light sources (130, 131, 132, 133) include a first light source (131) configured to emit light of a first wavelength range so that blood vessels can be marked by a first fluorescent material, and a second light source (132) configured to emit light of a second wavelength range so that a glioma can be marked by a second fluorescent material, and the first and second light sources are selectively controllable by the controller (140);
**characterized in that** the system further comprises a fluorescent multi-image acquirer (200) configured to acquire an actual image of a target part, a fluorescent vascular image (310) of blood vessels marked by the first fluorescent material, and a fluorescent glioma image (410) of a glioma marked by the second fluorescent material;
a blood vessel extractor (500) configured to extract a vascular shape from the acquired fluorescent vascular image (310);
a glioma image processor (600) configured to process the acquired fluorescent glioma image (410) so that a boundary of the glioma can become distinct;
a fluorescent multi-image aligner (700) configured to align the fluorescent vascular image (310) and the fluorescent glioma image (410) in real time; and
a fluorescent image display (800) configured to display a fluorescent multi-image in which the fluorescent vascular image (310) and the fluorescent glioma image (410) are aligned,
wherein the fluorescent multi-image aligner (700) is configured to measure pixel value-based similarities of a moving image which is the fluorescent vascular image (310) or the fluorescent glioma image (410) with respect to a fixed image which is the actual image and to select the transformation parameters corresponding to the highest similarity to transform the fluorescent vascular image (310) or the fluorescent glioma image (410) to match the actual image using [Equation 1];
wherein the transformation parameters comprise a scale factor, a radian-based angle, x and y values of a center position (x, y) of the fluorescent vascular image (310) or the fluorescent glioma image (410) after transformation, and translated x and y values (x', y'):

[Equation 1]

$$\begin{bmatrix} x' \\ y' \end{bmatrix} = \begin{bmatrix} \lambda & 0 \\ 0 & \lambda \end{bmatrix} \begin{bmatrix} \cos\theta & -\sin\theta \\ \sin\theta & \cos\theta \end{bmatrix} \begin{bmatrix} x - C_x \\ y - C_y \end{bmatrix} + \begin{bmatrix} T_x + C_x \\ T_y + C_y \end{bmatrix}$$

wherein $\lambda$ is a scale factor, $\theta$ is a rotation angle, $(C_x, C_y)$ are values of a rotation center position, and $(T_x, T_y)$ are values of translated elements.

2. The system of claim 1, wherein the light sources (130, 131, 132, 133) include filters,
   wherein the first light source (131) includes a first filter, and
   the second light source (132) includes a second filter.

3. The system of claim 1, wherein the blood vessels to be imaged are to be marked to fluoresce due to indocyanine green (ICG), and
   the boundary of a glioma to be imaged s to be marked to fluoresce due to one of 5-aminolevulinic acid (5-ALA) and protoporphyrin IX (PpIX) converted from 5-ALA.

4. The system of claim 1, wherein the light sources (130, 131, 132, 133) further include a third light source (133) configured to emit white light.

5. The system of claim 1, wherein the first wavelength range is 750 nm to 800 nm, and
   the second wavelength range is 350 nm to 450 nm.

6. The system of claim 1, wherein the probe (110) is formed of a flexible material.

7. The system of claim 1, wherein the fluorescent multi-image acquirer (200) includes:

   a fluorescent vascular shape image acquirer (300) configured to acquire the fluorescent vascular image (310) in which positions of the imaged blood vessels marked by the first fluorescent material are marked; and
   a fluorescent glioma image acquirer (400) configured to acquire the fluorescent glioma image (410) in which a position of the imaged glioma marked by the second fluorescent material is marked.

8. The system of claim 1, wherein the fluorescent image display (800) is configured to display at least one of the actual image, the fluorescent vascular image (310), the fluorescent glioma image (410), and the fluorescent multi-image according to a selection of an operator or a user.

9. The system of claim 8, wherein the fluorescent image display (800) is configured to set individual opacity values of the actual image, the fluorescent vascular image (310), the fluorescent glioma image (410), and the fluorescent multi-image to simultaneously display a plurality of images overlapping each other, and to adjust the opacity values of the individual images to display only a specific image as necessary.

10. The system of claim 1, wherein the pen-shaped medical fluorescent imaging device (100) includes a transmitter configured to transmit the actual image, the fluorescent vascular image (310), and the fluorescent glioma image (410) captured by the image capturing section (120) to the fluorescent multi-image acquirer (200) through wireless communication.

**Patentansprüche**

1. System zur Aufnahme mehrerer Fluoreszenzbilder, wobei das System umfasst:

   eine stiftförmige medizinische Fluoreszenzbildgebungsvorrichtung (100), umfassend eine Sonde (110), die konfiguriert ist, so dass sie sich in Längsrichtung erstreckt und an einem Ende einen Bilderfassungsabschnitt (120) enthält;
   eine Vielzahl von Lichtquellen (130, 131, 132, 133), die konfiguriert sind, so dass sie angeordnet sind, um den Bilderfassungsabschnitt (120) zu umgeben;
und eine Steuerung (140), die konfiguriert ist, um die Lichtquellen (130, 131, 132, 133) zu steuern,
wobei die Lichtquellen (130, 131, 132, 133) eine erste Lichtquelle (131), die konfiguriert ist, um Licht eines ersten Wellenlängenbereichs zu emittieren, so dass Blutgefäße durch ein erstes Fluoreszenzmaterial markiert werden können, und eine zweite Lichtquelle (132), die konfiguriert ist, um Licht eines zweiten Wellenlängenbereichs zu emittieren, so dass ein Gliom durch ein zweites Fluoreszenzmaterial markiert werden kann, enthalten und die erste und zweite Lichtquelle durch die Steuerung (140) selektiv steuerbar sind;
dadurch gekennzeichnat, dass das System ferner umfasst:

eine Fluoreszenzmehrfachbild-Aufnahmevorrichtung (200), die konfiguriert ist, um ein tatsächliches Bild eines Objektteils, ein fluoreszierendes Gefäßbild (310) von Blutgefäßen, die durch das erste Fluoreszenzmaterial markiert sind, und ein fluoreszierendes Gliom-Bild (410) von einem Gliom, das durch das zweite Fluoreszenzmaterial markiert ist, aufzunehmen;
einen Blutgefäßextraktor (500), der konfiguriert ist, um eine Gefäßform aus dem aufgenommenen fluoreszierenden Gefäßbild (310) zu extrahieren;
einen Gliombildprozessor (600), der konfiguriert ist, um das aufgenommene fluoreszierende Gliombild (410) so zu verarbeiten, dass eine Grenze des Glioms deutlich werden kann;
eine Fluoreszenzmehrfachbild-Ausrichtungsvorrichtung (700), die konfiguriert ist, um das fluoreszierende Gefäßbild (310) und das fluoreszierende Gliombild (410) in Echtzeit auszurichten; und
eine Fluoreszenzbildanzeige (800), die konfiguriert ist, um ein Fluoreszenzmehrfachbild anzuzeigen, in dem das fluoreszierende Gefäßbild (310) und das fluoreszierende Gliombild (410) ausgerichtet sind,

wobei die Fluoreszenzmehrfachbild-Ausrichtungsvorrichtung (700) konfiguriert ist, um pixelwertbasierte Ähnlichkeiten eines bewegten Bildes, welches das fluoreszierende Gefäßbild (310) oder das fluoreszierende Gliombild (410) ist, in Bezug auf ein festes Bild, welches das tatsächliche Bild ist, zu messen und die Transformationsparameter, die der höchsten Ähnlichkeit entsprechen, auszuwählen, um das fluoreszierende Gefäßbild (310) oder das fluoreszierende Gliombild (410) so zu transformieren, dass es mit dem tatsächlichen Bild unter Verwendung von [Gleichung 1] übereinstimmt;
wobei die Transformationsparameter einen Skalierungsfaktor, einen Winkel im Bogenmaß, x- und y-Werte einer Mittelposition (x, y) des fluoreszierenden Gefäßbildes (310) oder des fluoreszierenden Gliombildes (410) nach der Transformation und übersetzte x- und y-Werte (x', y') umfassen:

[Gleichung 1]

$$\begin{bmatrix} x' \\ y' \end{bmatrix} = \begin{bmatrix} \lambda & 0 \\ 0 & \lambda \end{bmatrix} \begin{bmatrix} \cos\theta & -\sin\theta \\ \sin\theta & \cos\theta \end{bmatrix} \begin{bmatrix} x - C_x \\ y - C_y \end{bmatrix} + \begin{bmatrix} T_x + C_x \\ T_y + C_y \end{bmatrix}$$

wobei $\lambda$ ein Skalierungsfaktor ist, $\theta$ ein Drehwinkel ist, $(C_x, C_y)$ Werte einer Rotationsmittelposition sind und $(T_x, T_y)$ Werte der übersetzten Elemente sind.

2. System nach Anspruch 1, wobei die Lichtquellen (130, 131, 132, 133) Filter umfassen, wobei die erste Lichtquelle (131) einen ersten Filter enthält und die zweite Lichtquelle (132) einen zweiten Filter enthält.

3. System nach Anspruch 1, wobei die abzubildenden Blutgefäße zu markieren sind, so dass sie aufgrund von Indocyaningrün (ICG) fluoreszieren und die Grenze eines abzubildenden Glioms zu markieren ist, so dass sie aufgrund eines von 5-Aminolevulinsäure (5-ALA) und Protoporphyrin IX (PpIX), welches aus 5-ALA umgewandelt wurde, fluoresziert.

4. System nach Anspruch 1, wobei die Lichtquellen (130, 131, 132, 133) ferner eine dritte Lichtquelle (133) enthalten, die konfiguriert ist, um weißes Licht zu emittieren.

5. System nach Anspruch 1, wobei der erste Wellenlängenbereich 750 nm bis 850 nm ist und der zweite Wellenlängenbereich 350 nm bis 450 nm ist.

**6.** System nach Anspruch 1, wobei die Sonde (110) aus einem flexiblen Material geformt ist.

**7.** System nach Anspruch 1, wobei die Fluoreszenzmehrfachbild-Aufnahmevorrichtung (200) enthält:

eine Fluoreszenzgefäßformbild-Aufnahmevorrichtung (300), die konfiguriert ist, um das fluoreszierende Gefäßbild (310), in dem Positionen der durch das erste Fluoreszenzmaterial markierten abgebildeten Blutgefäße markiert sind, aufzunehmen; und

eine Fluoreszenzgliombild-Aufnahmevorrichtung (400), die konfiguriert ist, um das fluoreszierende Gliombild (410), in dem eine Position des durch das zweite Fluoreszenzmaterial markierten abgebildeten Glioms markiert ist, aufzunehmen.

**8.** System nach Anspruch 1, wobei die Fluoreszenzbildanzeige (800) konfiguriert ist, um mindestens eines des tatsächlichen Bildes, des fluoreszierenden Gefäßbildes (310), des fluoreszierenden Gliombildes (410) und des fluoreszierenden Mehrfachbildes nach einer Auswahl eines Bedieners oder eines Benutzers anzuzeigen.

**9.** System nach Anspruch 8, wobei die Fluoreszenzbildanzeige (800) konfiguriert ist, um einzelne Opazitätswerte des tatsächlichen Bildes, des fluoreszierenden Gefäßbildes (310), des fluoreszierenden Gliombildes (410) und des fluoreszierenden Mehrfachbildes einzustellen, um gleichzeitig mehrere sich überlappende Bilder anzuzeigen, und die Opazitätswerte der einzelnen Bilder anzupassen, um nur ein bestimmtes Bild nach Bedarf anzuzeigen.

**10.** System nach Anspruch 1, wobei die stiftförmige medizinische Fluoreszenzbildgebungsvorrichtung (100) einen Sender enthält, der konfiguriert ist, um das tatsächliche Bild, das fluoreszierende Gefäßbild (310) und das fluoreszierende Gliombild (410), die durch den Bilderfassungsabschnitt (120) erfasst wurden, an die Fluoreszenzmehrfachbild-Aufnahmevorrichtung (200) durch drahtlose Kommunikation zu übertragen.


**Revendications**

**1.** Système d'acquisition d'images fluorescentes multiples, le système comprenant:

le dispositif d'imagerie médicale fluorescente en forme de stylo (100) comprenant une sonde (110) configurée pour s'étendre dans une direction longitudinale et inclure une section de capture d'image (120) à une extrémité; une pluralité de sources de lumière (130, 131, 132, 133) configurées pour être disposées de manière à entourer la section de capture d'image (120) ; et un dispositif de commande (140) configuré pour commander les sources de lumière (130, 131, 132, 133), dans lequel les sources de lumière (130, 131, 132, 133) comprennent une première source de lumière (131) configurée pour émettre de la lumière d'une première plage de longueurs d'onde de sorte que les vaisseaux sanguins peuvent être marqués par un premier matériau fluorescent, et une deuxième source de lumière (132) configurée pour émettre de la lumière d'une deuxième plage de longueurs d'onde de sorte qu'un gliome peut être marqué par un deuxième matériau fluorescent, et les première et deuxième sources de lumière peuvent être commandées sélectivement par le dispositif de com-mande (140); **caractérisé en ce que** le système comprend en outre un dispositif d'acquisition multi-image fluorescent (200) configuré pour acquérir une image réelle d'une partie cible, une image vasculaire fluorescente (310) de vaisseaux sanguins marquée par le premier matériau fluorescent, et une image de gliome fluorescente (410) d'un gliome marqué par le deuxième matériau fluorescent;

un extracteur de vaisseaux sanguins (500) configuré pour extraire une forme vasculaire à partir de l'image vasculaire fluorescente acquise (310);

un processeur d'images de gliome (600) configuré pour traiter l'image fluorescente acquise du gliome (410) pour distinguer une limite du gliome;

un aligneur multi-image fluorescent (700) configuré pour aligner l'image vasculaire fluorescente (310) et l'image fluorescente du gliome (410) en temps réel; et

un affichage d'image fluorescente (800) configuré pour afficher une multi-image fluorescente dans laquelle l'image vasculaire fluorescente (310) et l'image fluorescente du gliome (410) sont alignées, dans lequel l'aligneur multi-image fluorescent (700) est configuré pour mesurer des similitudes fondées sur la valeur des pixels de l'image vasculaire fluorescente (310) ou de l'image de gliome fluorescente (410) par rapport à une image fixe qui est l'image réelle et pour sélectionner les paramètres de transformation sont utilisés pour transformer l'image vasculaire fluorescente (310) ou l'image de gliome fluorescente (410) pour correspondre à l'image réelle en utilisant [l'équation 1]; dans lequel les paramètres de transformation comprennent un facteur d'échelle, un angle fondé sur le radian, les valeurs x et y d'une position centrale (x, y) de l'image vasculaire fluorescente (310) ou de l'image fluorescente du gliome (410) après transformation, et les valeurs x et y traduites (x', y'):

[Equation 1]

$$\begin{bmatrix} x' \\ y' \end{bmatrix} = \begin{bmatrix} \lambda & 0 \\ 0 & \lambda \end{bmatrix} \begin{bmatrix} \cos\theta & -\sin\theta \\ \sin\theta & \cos\theta \end{bmatrix} \begin{bmatrix} x - C_x \\ y - C_y \end{bmatrix} + \begin{bmatrix} T_x + C_x \\ T_y + C_y \end{bmatrix}$$

où $\lambda$ est un facteur d'échelle, $\theta$ est un angle de rotation, $(C_x, C_y)$ sont des valeurs d'une position de centre de rotation, et $(T_x, T_y)$ sont des valeurs d'éléments traduits.

2. Système selon la revendication 1, dans lequel les sources de lumière (130, 131, 132, 133) comprennent des filtres, dans lequel la première source de lumière (131) comprend un premier filtre, et la deuxième source lumineuse (132) comprend un deuxième filtre.

3. Système selon la revendication 1, dans lequel les vaisseaux sanguins à imager doivent être marqués pour être fluorescents en raison du vert d'indocyanine (ICG), et la limite d'un gliome à imager est marquée pour devenir fluorescente en raison de l'acide 5-aminolevulinique (5-ALA) et de la protoporphyrine IX (PpIX) convertis à partir du 5-ALA.

4. Système selon la revendication 1, dans lequel les sources de lumière (130, 131, 132, 133) comprennent en outre une troisième source de lumière (133) configurée pour émettre de la lumière blanche.

5. Système selon la revendication 1, dans lequel la première plage de longueurs d'onde est de 750 nm à 800 nm, et la deuxième plage de longueur d'onde est de 350 nm à 450 nm.

6. Système selon la revendication 1, dans lequel la sonde (110) est formée d'un matériau flexible.

7. Système selon la revendication 1, dans lequel le dispositif d'acquisition multi-images fluorescent (200) comprend:

un dispositif d'acquisition d'images vasculaires fluorescentes (300) configuré pour acquérir l'image vasculaire fluorescente (310) dans laquelle sont marquées les positions des vaisseaux sanguins imagés marqués par le premier matériau fluorescent; et
un dispositif d'acquisition d'image de gliome fluorescent (400) configuré pour acquérir l'image de gliome fluorescent (410) dans lequel une position du gliome imagé marquée par le deuxième matériau fluorescent est marquée.

8. Système selon la revendication 1, dans lequel l'affichage d'image fluorescente (800) est configuré pour afficher au moins une de l'image réelle, l'image vasculaire fluorescente (310), l'image fluorescente de gliome (410) et les images fluorescentes multiples selon une sélection d'un opérateur ou d'un utilisateur.

9. Système selon la revendication 8, dans lequel l'affichage d'images fluorescentes (800) est configuré pour définir des valeurs d'opacité individuelles de l'image réelle, de l'image vasculaire fluorescente (310), de l'image fluorescente de gliome (410) et de l'image fluorescente multiple pour afficher simultanément une pluralité d'images se chevauchant les unes les autres, et pour ajuster les valeurs d'opacité des images individuelles afin d'afficher seulement une image spécifique si nécessaire.

10. Système selon la revendication 1, dans lequel le dispositif d'imagerie médicale fluorescent en forme de stylo (100) comprend un émetteur configuré pour transmettre l'image réelle, l'image vasculaire fluorescente (310) et l'image fluorescente de gliome (410) capturée par la section de capture d'image (120) au dispositif d'acquisition multi-image fluorescente (200) par communication sans fil.

[Fig. 1]

[Fig. 2]

130

131

133

133

120

132

EP 3 332 689 B1

[Fig. 3]

**100**

ACQUIRE IMAGE

**310**

ICG

**410**

5-ALA

EXTRACT BLOOD VESSELS
AND SHARPEN IMAGE

INGEST 5ALA

INTRAVENOUSLY
INJECT ICG

**510**

**610**

ALIGN IMAGES IN REAL TIME

**810**

OUTPUT ON DISPLAY

[Fig. 4]

EP 3 332 689 B1

**EP 3 332 689 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011184243 A1